# EUROPEAN PATENT SPECIFICATION

(11) **EP 4 389 043 B1**
(45) Date of publication and mention of the grant of the patent: **25.06.2025**
(21) Application number: 23217854.1
(22) Date of filing: 19.12.2023
(51) Int. Cl.: A61B 18/12

(54) **ELECTROSURGICAL GENERATOR WITH DUAL OUTLETS**
ELEKTROCHIRURGISCHER GENERATOR MIT ZWEI AUSGÄNGEN
GÉNÉRATEUR ÉLECTROCHIRURGICAL À DEUX SORTIES

(30) Priority: 23.12.2022 US 202263435060 P
(43) Date of publication of application: 26.06.2024
(73) Proprietor: Olympus Winter & Ibe GmbH, 22045 Hamburg (DE)
(72) Inventor: Dijkstra, Jelle, 12205 Berlin (DE); Janich, Fabian, 14469 Potsdam (DE); Fähsing, Thomas, 12305 Berlin (DE); Ramin, Daniel, 14558 Nuthetal (DE)
(74) Representative: Glawe, Delfs, Moll

(56) References cited:
- DE-A1- 102005 044 918
- DE-B3- 102013 005 277
- US-A1- 2015 282 860
- US-A1- 2019 132 062
- US-A1- 2021 361 337
- US-A1- 2022 192 726
- US-A1- 2023 069 525

## Description

The invention relates to an electrosurgical generator configured to output a high-frequency alternating voltage to at least one electrosurgical instrument. The electrosurgical generator comprises an inverter unit generating high-frequency alternating voltage to be supplied to at least two output sockets configured for simultaneous connection of electrosurgical instruments.

In electrosurgery or high-frequency surgery, an electrosurgical instrument such as an electroscalpel is used to apply high-frequency alternating current to tissue in the human body. Usually high-frequency in the radiofrequency range of about 200 kHz to up to 4,000 kHz is used. This results in local heating of the tissue. Thereby, the tissue is cut or severed by heating, and the tissue is removed by thermal resection. A major advantage of this is that bleeding can be stopped at the same time as the cutting is made by closing the affected vessels, and electrosurgical instruments can be used for other applications, such as coagulation. Different kind of applications require different electrosurgical instruments.

For some applications it may be required to perform surgery tasks utilizing more than one, in particular two electrosurgical instruments at the same time (dual activation). In order to make this feasible, separate electrosurgical generators may be used, one for each electrosurgical instrument. A typical problem encountered is that interference between the outputs of the high-voltages generated by the generators may occur. Such interference may include an unwanted low-frequency beat similar to the phenomenon as encountered in the field of acoustics if two frequencies are close to each other but do not exactly match. Such low frequent beat is adverse to stable operating conditions of the electrosurgical instruments. This is especially true if two distinct electrosurgical generators are used but may also be encountered if a single electrosurgical generator having dual output stages and inverters is employed, thereby allowing dual activation of electrosurgical instruments. However, avoiding unwanted interference is still difficult.

Relevant prior art is disclosed in US 2022/192726 A1, US 2021/361337 A1, DE 10 2005 044918 A1, US 2015/282860 A1, US 2019/132062 A1, DE 10 2013 005277 B3 and US 2023/069525 A1.

It is thus an object of the invention to provide an improved electrosurgical generator allowing dual activation that reduces this drawback.

The solution according to the invention consists in an electrosurgical generator according to the features of the independent claim. Advantageous developments are the subject matter of the dependent claims.

In an electrosurgical generator configured to output a high-frequency alternating voltage to an electrosurgical instrument, comprising an inverter unit generating a high-frequency alternating voltage to be supplied to at least two output sockets for connection of electrosurgical instruments, wherein the inverter unit is configured for simultaneous activation of the output sockets and the respective connected electrosurgical instruments, it is provided according to the invention that the inverter unit is a multilevel inverter and comprises a plurality of co-operating inverter cells, said plurality of inverter cells being connected in a cascaded manner in a first state and being split into at least two groups in a second state, each group being assigned to one of the output sockets and feeding its generated high-frequency alternating output voltage via electrode lines to said assigned output socket, wherein the inverter unit is selectively switchable between first and second state upon direction and/or command.

Modern electrosurgical generators employ an inverter which is comprised of a plurality of inverter cells. The inverter cells are usually connected in series in order to provide a high HF voltage that is required for supplying the electrosurgical instruments. An electrosurgical generator comprising such an inverter is the subject of applicant's yet unpublished patent application filed as DE 10 2021 122 282. This allows for more efficient and versatile generation of the high frequency voltage. In particular, the generated voltage is determined by the reference signal used to control the inverter cells, thereby providing a positive control with respect to frequency, phase and optionally also shape of the generated AC voltage.

In the following, some expressions that are used within the context of the invention are explained:
The inverter unit is a device to provide the actual high-frequency alternating voltage output for the surgical instrument to be connected to the output socket. The inverter unit comprises a plurality of inverter cells which are connected with each other in order to supply the high-frequency high voltage necessary for the electrosurgical instruments. Frequency, phase and usually also amplitude of the high-frequency voltage generated by the inverter unit is controlled by a reference signal as used by the controller unit.

In the context of the present application the term "high-frequency" relates to frequencies in the radiofrequency range of 200 kHz to 4000 kHz as generated by the inverter of the electrosurgical generator. The high frequency alternating voltage is typically a high voltage. In the context of the present patent high voltage is considered as having an amplitudes in the high voltage range, in particular up to 10 kV, preferably up to 4000 Volt and further preferably more than 10 Volt.

The invention draws on the fact that employing an inverter being configured as a stacked multi-cell inverter allows to deliberately split the plurality of inverter cells into at least two distinct group, one group each feeding one output. The plurality of inverter cells and therefore the distinct groups are under common control, thereby allowing for a simultaneous controlling of the distinct groups and the high-voltage output as provided by each group. Thereby, unwanted interference patterns can be avoided. As a result, an uncontrolled low-frequency beat that has adversely affected common activation of two or more electrical surgical instruments by employing the conventional approach can be largely suppressed or even eliminated. Thereby, thermal energy and cutting power of the electrosurgical instrument can be controlled much better, thereby increasing quality of the work performed by the surgeon by the electrosurgical instruments.

All these benefits can be realized by selecting the second state. This may happen preferably according to a user command and/or to a direction issued by the main control unit and/or a mode of electrosurgical generator. However, by selecting the first state the electrosurgical generator is enabled to operate the inverter cells as a single group, thereby functioning as a conventional multilevel generator being capable of an extra fine gradation of the output voltage.

Therefore the invention achieves at least two major benefits for the patient, reducing hazardous conditions and improving quality of the surgery. Furthermore, versatility of the electrosurgical generator is enhanced.

Preferably, the at least two groups with their respective inverter cells are being commonly controlled by synchronized reference signals being generated by a control unit. In the context of the present invention the term synchronized means having the same frequency, and optionally also same phase.

Employing an inverter using multi-cell technology having the cells split into distinct groups under common control brings further the advantage that frequency is stabilized even under varying load conditions. This applies to either output, thereby keeping them in tied together even in difficult conditions, in particular but not limited to situations in which inhomogenic tissue is to be treated where electrical impedance varies greatly. Conventionally this would often lead to frequency deviations and therefore uncontrolled beat frequencies with detrimental effects on quality of the electrosurgical operation. All this can be effectively avoided due to common control of the groups of inverter cells as claimed.

Advantageously, the reference signals are being synchronized such that the output voltages have the same frequency. By providing such synchronized reference signals for the groups of inverter cells it is ensured that the voltages generated by the groups of inverter cells are in synchronism, too. Such a synchronous frequency brings the advantage that there is no difference in frequency between the high-frequency voltage generated by one group and the high-frequency voltage generated by another group. If both waveforms have the exact same frequency any low frequency interference pattern can be effectively avoided. In particular, due to the frequency being identical there is no risk of creating a low beat condition which requires by definition presence of a frequency difference (which then determines the beat frequency).

Further preferably, the output of one group is synchronized in phase with a predefined phase shift to the output voltage of another group. Thereby, not only frequency becomes synchronized but also phase becomes synchronized between the output of one group with respect to the output of another group. The predefined phase shift may be zero so that the outputs voltage of different groups will have same frequency and same phase. However, this is not necessarily so, another preferred option is to have a phase shift of 180°, i.e. reversed polarity. Thereby the output voltage can be inverted with respect to the output voltage of another group. Surprisingly, there are certain situations in which inverted polarity provides improved interference characteristics.

In an advantageous embodiment, at least two groups share one of the electrode lines, in particular they share a common neutral electrode. By virtue of this, the number of electrodes to be applied to the body of the patient can be reduced. If it is the common neutral electrode, then it is ensured that those instruments have the same neutral potential thereby reducing unwanted cross-currents at the neutral potential.

Preferably, the output sockets are configured for unipolar electrosurgical instruments. By virtue of this, two unipolar electrosurgical instruments can be supplied with HF power, wherein preferably a shared a neutral electrode is used. Such a configuration is beneficial as it allows for dual independent activation of both unipolar electrosurgical instruments while just requiring one additional electrode at the body of the patient. However, as far as further operation, in particular regulation of power, is concerned some interdependencies between outputs to the respective electrosurgical instrument exist. By providing suitable control systems such interdependencies can be dealt with to a large extent.

In a preferred alternative embodiment, each group has its own electrodes, in particular an active and neutral electrode, neither of them being shared with another group. Thereby, the attached electrosurgical instruments become more independent since each of them has its own return (neutral) electrode. It is further possible to provide a preferable galvanic isolation. This enables a more independent application of the electrosurgical instrument of one group with respect to the electrosurgical instrument of another group.

In a particularly advantageous embodiment the output sockets are configured for bipolar electrosurgical instruments. Thereby dual activation of bipolar electrosurgical instruments can be achieved. However, even though both instruments are fully galvanically isolated, cross-currents may flow between the electrosurgical instruments due to the patient body's tissue forming a closed loop between the two electrosurgical instruments. It was found that the amplitude of the cross-currents depend on the relative polarity of the voltage supplying electrosurgical instrument. Surprisingly, a minimization of the cross-current can be achieved by selectively changing a polarity of the output voltage of one of the groups. By virtue of this, with rather simple means unwanted cross-current can be reduced.

In a preferred embodiment, a current monitor is provided which is configured to determine a cross-current between the output sockets. Thereby it can be detected when a cross-current occurs. This can be employed for example by the control unit to monitor that the cross-current does not become excessive and therefore protective function can be realized.

Advantageously, an automatic polarity switcher is provided for at least one of the groups, wherein the automatic polarity switcher is configured to co-operate with the current monitor for identifying a polarity having minimal cross-current. This enables selection of a polarity such that the cross-current is minimized and therefore unwanted interferences can be suppressed. In a preferred embodiment, the automatic polarity switcher is configured to co-operate with the current monitor such as to (i) determine the cross-current in a first polarity state, (ii) then switch the polarity to a second polarity state and determine the cross-current in the second polarity state, (iii) comparing the cross-currents of the first and second polarity state, (iv) determine which polarity state has a lower cross-current, and (v) selecting the polarity having the lower cross-current. This can be performed manually or, preferably, automatically so that the polarity of at least one of the output voltage is selected such as to minimize cross-current and therefore interferences. A major advantage of the automatic polarity switcher is that it automatically keeps track to maintain the lower cross-current scenario and therefore provides a valuable boost of practicability of the dual activated electrosurgical instruments.

Preferably, a beat frequency monitor is provided that is configured to determine a beat frequency between outputted high-frequency voltages of the at least two output sockets. Thereby it can be monitored of whether the frequencies are properly tied, particularly whether synchronism is achieved and maintained and the at least two electrosurgical instruments attached to the output sockets can be operated safely. If an unwanted beat frequency occurs then it is an indicator that frequency synchronism needs to be readjusted. To this end, the beat frequency monitor preferably co-operates with a synchronizing unit for ensuring frequency, and preferably phase, synchronicity between the at least two groups. Thereby synchronicity in terms of frequency, and if applicable also phase, can be maintained. Preferably, numerically controlled oscillators (NCO) and/or PLL circuits may be employed for this purpose.

Advantageously, a decoupling control unit is provided that is configured to automatically modify a power setting of one of the group of the inverter cells upon a change of a power setting of an other group of the inverter cells. Preferably, said decoupling control unit is part of or interacting with the common control unit. Thereby, interdependencies that may exist between the groups of inverters can be automatically managed. This provides for a much improved user experience in particular in situations in which changing settings of one group also affects the other group. For example, changing of power delivered by one group of the inverter cells to one of the instruments also leads to a change in power delivered by the other group of inverters to the other instrument, and vice versa. This is largely due to the instruments working on and therefore being electrically connected to the same patient body. This cannot be eliminated due to the laws of physics, however according to this advantageous aspect of the invention it was realized that said interdependence can be controlled. To this end, the decoupling control unit is preferably configured to provide a control structure reversing the physical coupling. By virtue of such a decoupling control unit the system behaves like two independent sub-systems from a control point of view. This enables the surgeon operating the electrosurgical generator to just change the power delivered to one of the instruments, and the decoupling control unit automatically performs the necessary adjustments to the groups of inverter cells in order to achieve the desired power change. The decoupling control unit may further be configured to comprise a predictive parameter calculation sub-device. It is configured to pre-calculate necessary adjustments for e.g. amplitude and/or phase of either of the group of inverters. Thereby, a stable control setting can be set quickly without the need of iterations of re-setting. This further improves controllability and control dynamics.

In an advantageous embodiment a switching device is provided which is configured to selectively alter an electrode, in particular a neutral electrode, between a shared and an unshared state. This enables a quick change of configuration according to need. If required for operation or, in particular in the case of unipolar electrosurgical instruments, the neutral electrodes may be switched into a shared state. However, in other configurations, in particular but not limited to usage of bipolar instruments, it may be beneficial to have a neutral electrode in an unshared state for more independent operation.

The invention is explained in more detail below by way of examples in conjunction with the accompanying drawing showing advantageous embodiments. In the drawing:
- Fig. 1: shows a frontal view of an electrosurgical generator with two attached electrosurgical instruments;
- Fig. 2: shows a schematic functional diagram of the electrosurgical generator including two output sockets;
- Fig. 3A, B: shows a plurality of inverted cells in a first state and in a second state being split in groups;
- Fig. 4: shows an internal configuration of two inverter cells;
- Fig. 5A, B: show an equivalent circuit diagram and currents for unipolar electrosurgical instruments;
- Fig. 6A, B: shows another equivalent circuit diagram and currents for unipolar electrosurgical instruments; and
- Fig. 7A, B: shows an equivalent circuit diagram and currents for bipolar electrosurgical instruments.

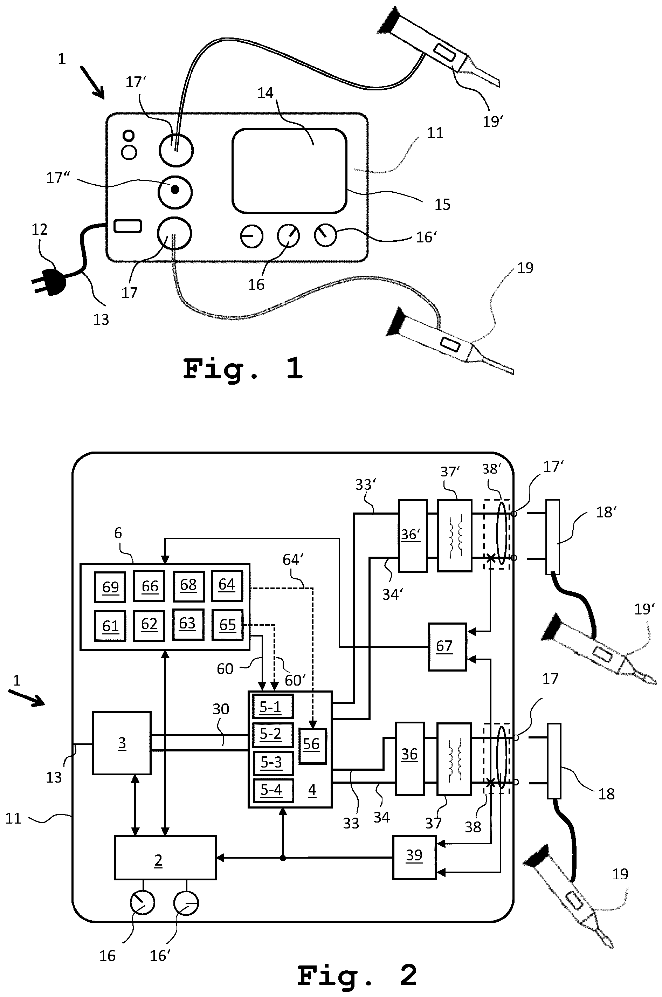

An electrosurgical generator according to an exemplary embodiment of the invention is illustrated in Fig. 1. The electrosurgical generator, identified as a whole by reference numeral 1, comprises a housing 11 having at least two output sockets 17, 17' (and optionally an additional output socket 17") for connection of electrosurgical instruments 19, 19'. A power supply cable 13 is provided with a plug 12 for connection to an electrical power source (not shown) which may be an electricity grid, like AC mains in a building, or an off-grid source of electric energy, like a 12 Volt or 24 Volt battery in a vehicle or mobile hospital. Further, a user interface 14 is provided comprising a display 15 which may be a touchscreen, or knobs 16, 16' may be present for inputs by a user.

Further referring to Fig. 1 and Fig. 2, said electrosurgical instrument 19 comprises a cable with a plug 18 which is to be plugged-in into the output socket 17 in order to supply high-frequency alternating voltage for the electrosurgical instrument 19. This applies likewise with respect to the other electrosurgical instrument 19' to be connected by its plug 18' to the output socket 17'.

Fig. 2 shows a schematic functional diagram of the electrosurgical generator. Electric power is delivered by the power supply cable 13 to a power supply unit 3 of the electrosurgical generator 1. The power supply unit provides electrical power, in the depicted embodiment DC power, to the various components, units and modules of the electrosurgical generator 1. In particular, it provides electrical power via a DC bus 30 to an inverter unit 4 configured for generating high-frequency alternating voltage, said high-frequency voltage being fed via a filter 36, a transformer 37 and sensor arrangement 38 for voltage and current measurement to the output socket 17 for supplying the plugged-in electrosurgical instrument 19. The high-voltage is usually in a range of a few kilovolts, but may have amplitudes in a range of several 10 Volt up to 4000 Volt. Further, the inverter unit 4 is provided with a second branch for outputting voltage in a likewise fashion via a filter 36', step-up transformer 37' and sensor arrangement 38' to the other output socket 17'.

Operation of the inverter unit 4 is governed by a master controller 2 which in turn is connected with the user interface 14 such that the user can issue directions and commands for operation of the electrosurgical generator 1. The master controller 2 generates corresponding control signals and governs the relevant components, units and modules of the electrosurgical generator 1 according to these instructions and commands. In particular, the master controller 2 provides command signals to a common control unit 6 which in turn controls the inverter unit 4 with its inverter cells, in the depicted exemplary embodiment four inverter cells 5-1, 5-2, 5-3, 5-4 are provided. The high-frequency voltage as generated is output by the inverter unit 4 to two electrode lines comprising an active electrode 33 and a neutral electrode 34 leading, via the filter 36, the step-up transformer 37 and the sensor arrangement 38 to the output socket 17. Likewise, a second high-frequency voltage is generated by the inverter unit 4 and supplied by means of two electrode lines comprising an active electrode 33' and a neutral electrode 34' leading, via filter 36', the step-up transformer 37' and the sensor arrangement 38' to the output socket 17'.

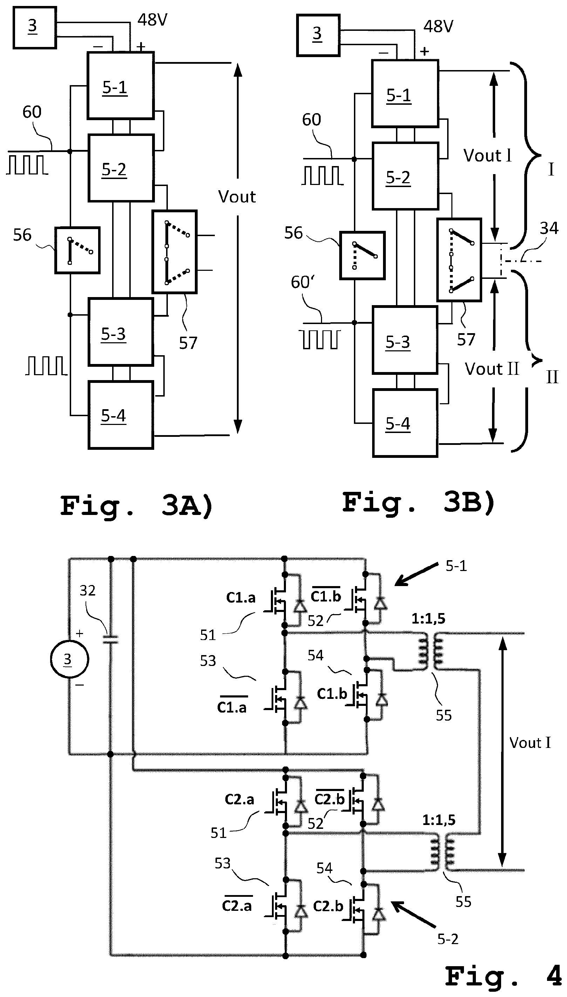

Generation of the high-frequency voltage by the inverter unit 4 and supplying to the output socket 17 or to the output sockets 17, 17', as the case may be, will be explained in the following with reference to Fig. 2 and Fig. 3A, B. As shown, the inverter unit 4 comprises a plurality of inverter cells 5-1, 5-2, 5-3, 5-4 which are driven by the common control unit 6. The inverter cells 5-1, 5-2, 5-3, 5-4 are being fed by DC voltage from the power supply unit 3, in the depicted case a DC voltage of 48 V. This DC voltage is applied to all of the inverter cells 5-1, 5-2, 5-3, 5-4. The inverter cells 5-1, 5-2, 5-3, 5-4 are provided with reference signals being generated by the common control unit 6 and conveyed to the inverter cells 5-1, 5-2, 5-3, 5-4 by means of a signal line 60.

The inverter cells 5-1, 5-2, 5-3, 5-4 2 can be connected in a selective manner. As shown in Fig. 3A, in a first state the inverter cells are connected in series to form a stacked inverter cell configuration. To each of the inverter cells 5-1, 5-2, 5-3, 5-4 DC voltage of, e.g. 48 Volt, is supplied by the power supply unit 3 and reference signals are applied to them as delivered by the line 60 from the common control unit 6 and being forwarded to the individual inverter cells 5-1, 5-2, 5-3, 5-4 directly or via a (closed) group switch 56. Owing to their series configuration, the output voltage of each of the inverter cells 5-1, 5-2, 5-3, 5-4 are added to each other to form a common output voltage Vout that is equal to the sum of the output voltage of all inverter cells 5-1, 5-2, 5-3, 5-4. This is considered as being the first state, wherein the inverter cells 5-1, 5-2, 5-3, 5-4 cooperate in the manner to form a multilevel inverter.

In Fig. 4 a detail view of inverter cells 5-1, 5-2 and their co-operation is shown. The inverter cells 5-1 and 5-2 are in a cascaded arrangement and both are being supplied by the same DC voltage of exemplary 48 V from the power supply unit 3, as illustrated on the left side edge of Fig. 4. Further a stabilization capacitor 32 is provided. Hereby the two inverter cells 5-1 and 5-2 are supplied with stabilized DC voltage. Each of the inverter cells comprises four power switches that operate as current valves and are arranged in an H-bridge configuration. The power switches are power semiconductor switches, e.g. configured as IGBTs, MOSFETs or GaNFETs. Power switches 51, 53 are connected in series and form a first branch, power switches 52, 54 are likewise connected in series and form a second branch. Center taps of the two branches are guided out and connected to both ends of the primary winding of a transformer 55. The transformer 55 comprises a secondary winding which serves as an output of the respective inverter-cell. The two power switches 51, 53 are driven by a common signal C1.a, wherein the signal supplied to the power switch 53 is provided in an inverted form. The two power switches 52, 54 of the second branch are likewise driven by a current signal C1.b, wherein the signal is supplied to the power switch 52 in inverted form. The signals C1.a and C1.b are generated in a manner known per se. Operation is as follows: in the event of a HIGH signal of C1.a the power switch 51 is put into the on state and the power switch 53 is put into the off state, that is to say the first power branch applies a positive potential to the upper connection of the transformer 55 and eventually to the active electrode line 34. Accordingly, in the event of a HIGH signal of C2.b the power switch 54 is put into the on state by the power switch 52 is put into the off state. The second power branch thus applies a negative potential to the lower connection of the transformer 55 and eventually to the neutral electrode line 33.

Now returning to Fig. 3A, the first state is shown having the inverter cells 5-1, 5-2, 5-3, 5-4 connected in series in a conventional manner. The reference signal is supplied by line 60 and distributed to the inverter cells 5-1, 5-2, 5-3, 5-4.

According to the present invention, there is a second state selectable in which the inverter cells 5-1, 5-2, 5-3, 5-4 are being split into at least two groups. This is shown in Fig. 3B in which two groups I, II are depicted. The groups I, II can be isolated from each other with respect to reference signals by a first group switch 56 and with respect to output voltage by second group switch 57. Each group I, II generates an output voltage Vout I, VoutII which is fed via electrode lines to the respective output sockets 17, 17'. The reference signal being supplied by the common control unit 6 is conveyed to the inverter cells 5-1, 5-2 of the first group I via signal line 60, whereas the reference signal for the inverter cells 5-3, 5-4 of the second group II is supplied by the common control unit via signal line 60'. By virtue of the common control unit 6 the reference signal fed to the inverter cells 5-3, 5-4 of the second group II has the same frequency as the reference signal supplied to the inverter cells 5-1, 5-2 of the first group I, however it may have a different or - as shown - a reverse polarity.

In other words, the plurality of inverter cells 5-1, 5-2, 5-3, 5-4 are split into two groups, the first group I comprising inverter cells 5-1, 5-2 generating a first output voltage Vout I being supplied to the first output socket 17. Likewise the, the second group II comprises inverter cells 5-3, 5-4 generating a second output voltage Vout II being supplied to the second output socket 17'. Thereby, an own output voltage is supplied to either of the output socket 17, 17' and the respective electrosurgical instruments 19, 19' which can be used at the same time and independently from each other (dual activation). Operation of the inverter cells 5-1, 5-2 of the first group I is controlled by the reference signal conveyed via line 60, and operation of the inverter cells 5-3, 5-4 of the second group II is controlled by the reference signal conveyed via line 60' having the same frequency as reference signal conveyed via line 60.

The inverter unit 4 and its inverter cells 5-1, 5-2, 5-3, 5-4 are being controlled by the common control unit 6. The common control unit 6 communicates with the master controller 2 and acts in accordance with directions and command as issued by the master controller 2. The common control unit 6 comprises a reference signal generator 61 configured to generate a reference signal for driving the inverter unit 4. The reference signal determines in particular frequency, phase and usually also amplitude of the high-frequency voltage generated by the inverter unit and its inverter cells 5-1, 5-2, 5-3, 5-4.

Further, the common control unit 6 comprises a frequency following unit 62 and a phase shift unit 63. The frequency following unit 62 is configured to tie a frequency of a reference signal 60' supplied to the second group II of inverter cells 5-3, 5-4 to the frequency of the reference signal 60 supplied to the first group I of inverter cells 5-1, 5-2. Preferably, the frequency following unit 62 is configured to synchronize the frequency of the reference signal 60' to the frequency of the reference signal 60 with a preselectable phase shift. Thereby, it is ensured that the groups I, II of inverter cells 5-1, 5-2, 5-3, 5-4 are being driven at the same frequency so that no frequency difference materializes, and as a result no beat frequency occurs. In order to verify this condition a beat frequency monitor 66 is provided which is configured to determine the beat frequency and interacts with the frequency following unit 62, preferably such as to ensure synchronicity, in particular frequency synchronicity.

Likewise, the phase shift unit 63 adjusts the phase of one of the reference signals 60, 60' such that they have a predefined phase shift. The phase shift is preselectable either by the user and/or by the master controller 2. In many cases this predefined phase shift will be zero such that the reference signal 60, 60' will not only have the same frequency but also the same phase. Further, there may be instances where it is desirable to invert the reference signal 60' with respect to reference signal 60. To this end, a polarity inverter 65 is provided at the common control unit 6, preferably inverting the reference signal 60' for the second group II.

Further, the common control unit 6 comprises state switch 64. It is activated when the inverter changes from the first state operating all inverter cells 5-1, 5-2, 5-3, 5-4 uniformly in a conventional manner to the second state wherein the inverter cells 5-1, 5-2, 5-3, 5-4 are split into groups. Upon entering the second state, the state switch 64 operates via a signal line 64' group switches 56, 57 in order to isolate connections between the groups of inverter cells 5-1, 5-2, 5-3, 5-4, thereby enabling the groups I, II to be operated independently. Operation of group switch 56 is mandatory in order to enable independent control of both groups I, II. Operating of second group switch 57 is optional depending on whether the electrodes of both groups I, II shall be galvanically isolated from each other or whether they shall have a shared electrode, typically a shared neutral electrode as it is indicated by the dashed dotted lines in Fig. 3B.

Yet further, a decoupling control unit 68 may be provided. Although the groups of inverter cells I, II are isolated from each other this does not mean that there is no interdependence at all. Situations may be encountered in which e.g. a change in power delivered by one group I of the inverter cells also leads to a change in power delivered by the other group II of inverters, and vice versa, since the respective instrument 19, 19' to which the respective group of inverters I, II is connected are working on the same body. So, there is some interdependence. This cannot be eliminated due to the laws of physics, however the invention has realized that said interdependence can be controlled. Therefore, the decoupling control unit 68 is advantageously configured to provide a control structure reversing the physical coupling. Such control structures are generally known in the art e.g. for decoupling MIMO (multi-input multi-output) industrial processes. By virtue of such a special decoupling control unit 68 the system behaves like two independent sub-systems from a control point of view. This enables the surgeon operating the electrosurgical to just change the power delivered to one of the instruments 19, and the common control unit 6 with its decoupling control unit automatically performs the necessary adjustments to both groups of inverter cells I, II in order to achieve the desired power change to the selected instrument. The decoupling control unit 68 may further be configured to comprise a predictive parameter calculation sub-device. It is configured to pre-calculate necessary adjustments for e.g. amplitude and/or phase of either of the group of inverters I, II. Thereby, a stable control setting can be dialed-in quickly without the need of iterations of re-setting. This further improves control dynamics.

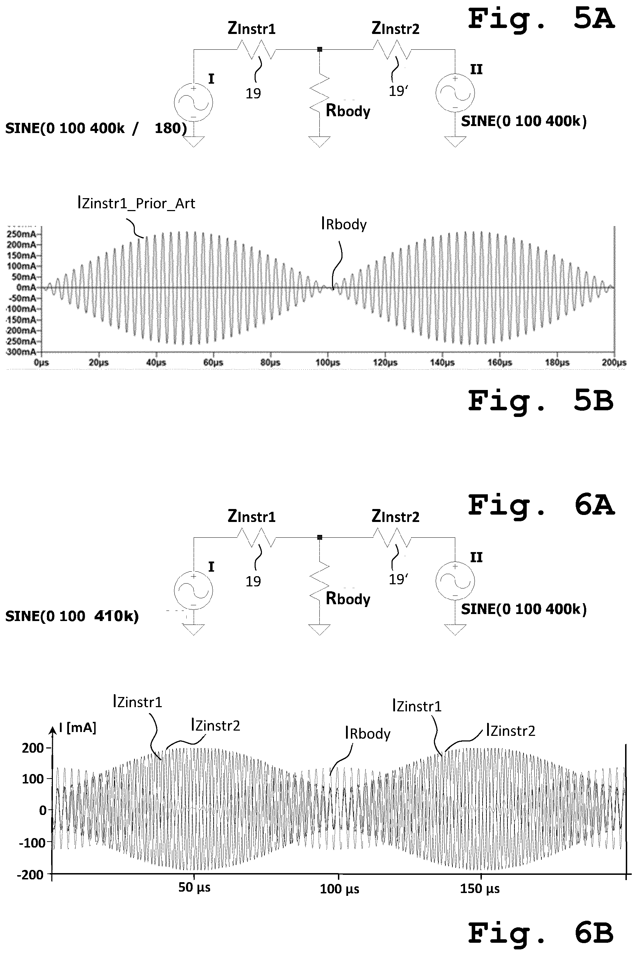

Reference is made to Fig. 5A, B showing an equivalent circuit diagram and currents for unipolar electrosurgical instruments. It relates to operation of the electrosurgical generator 1 with the inverter unit 4 in the second state configuration. The inverter cells 5-1, 5-2, 5-3, 5-4 are split into two groups I, II, wherein the output voltage provided by group I supplying electrosurgical instrument 19 is symbolized as a voltage source denoted by reference sign "I", and wherein the output voltage provided by group II supplying electrosurgical instrument 19' is symbolized as a voltage source denoted by reference sign "II". The body tissues surrounding electrosurgical instruments 19, 19' are being symbolized by an impedance Zinstr1, Zinstr2, respectively. Further, both unipolar electrosurgical instruments share a common neutral electrode connecting to the body of patient being symbolized by the resistance Rbody for the common neutral electrode path.

In Fig. 5B, the oscillating curve relates to the prior art. It shows the current flowing through electrosurgical instrument 19 (through Zinstr1). As it happens in the prior art, the voltage sources for both instruments have a slight and uncontrolled frequency difference, in the depicted case about 3%. The varying amplitude of the oscillating curve represents a resulting beat frequency which shows that an unwanted uncontrolled modulation occurs of the current flowing through said electrosurgical instrument 19. This adversely affects reliable operation of the instrument 19. - By synchronizing the frequency of the output voltages of groups I, II according to the present invention and inverting the reference signal for one of the groups by the polarity inverter 65 (or by setting a phase shift of 180° by means of the phase shift unit 63), there is no frequency difference and therefore no beat frequency occurs. Further due to said inverting the currents through both instruments 19, 19' cancel so that effectively the current flowing through the common electrode connecting to the patient body, symbolized by impedance Rbody, falls to zero (straight-line at 0 mA in Fig. 5B) . This allows for a much smoother operation of both electrosurgical instruments, and further effectively neutralizes most if not all currents through the common body electrode which further reduces current strain on the patient's body.

An equivalent circuit diagram for an alternative configuration for unipolar electrosurgical instruments is shown in Fig. 6A, B. Here, the phase shift is selected to be zero but the frequency for the inverter cells of group I is selected to a higher value, e.g. 410 kHz, than the frequency for the inverter cells of group II, e.g. 400 kHz. Accordingly, in this embodiment the frequencies of the two groups of inverter cells are tied to each other but are not identical. However, like in the foregoing embodiment both have the same amplitude. Other than that, the equivalent circuit diagram is the same as in the foregoing embodiment. In Fig. 6B, the larger oscillating curves labelled Izinstr1 and IZinstr2 relate to the currents flowing through the electrosurgical instruments 19, 19', respectively. It is to be noted that due to the rather similar frequencies these two larger oscillating curves labelled IZinstr1 and IZinstr2 appear to visually form a single curve. - The oscillating curve having the smaller amplitude represents the current flowing through the common electrode and the body of the patient, as represented by Rbody in the equivalent circuit diagram. Due to said frequency difference, a beat frequency occurs which is however well controlled and therefore manageable.

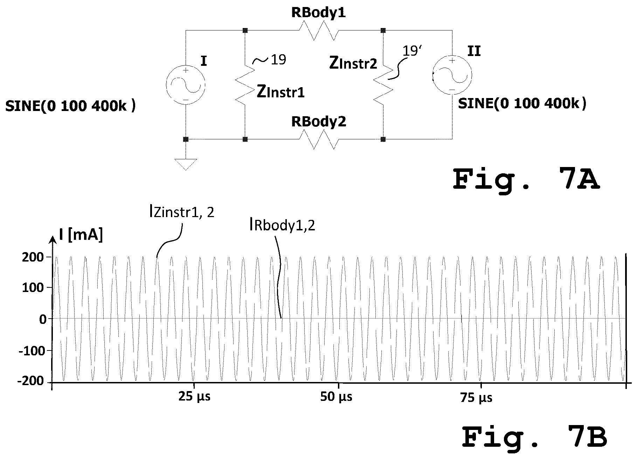

Reference is now made to Fig. 7A, B showing an equivalent circuit diagram and currents for bipolar electrosurgical instruments. Likewise to the scenario of Fig. 5A, B and Fig. 6A, B, it relates to operation of the electrosurgical generator 1 with the inverter unit 4 in the second state configuration. The inverter cells 5-1, 5-2, 5-3, 5-4 are split into two groups I, II, wherein the output voltage provided by group I supplying electrosurgical instrument 19 is symbolized as a voltage source denoted by reference sign "I", and wherein the output voltage provided by group II supplying electrosurgical instrument 19' is symbolized as a voltage source denoted by reference sign "II". Both output voltages have the same amplitude and are synchronized in frequency and phase. The body tissues surrounding electrosurgical instruments 19, 19' are being symbolized by an impedance Zinstr1, Zinstr2, respectively. Cross-currents may flow through the body of the patient between the locations of both instruments, and the respective impedances are symbolized by the impedances Rbody1 and Rbody2 in Fig. 7A.

Similar to Fig. 6B, in Fig. 7B the large oscillating curve relates to the current flowing through either electrosurgical instrument 19. 19'. By synchronizing according to the invention to the very same frequency, no frequency difference exists and no modulation occurs, eventually resulting in the beat frequency being absent. This has the positive effect of substantially reducing the amplitude of any cross-current flowing through the patient's body impedances Rbody1, 2 down to a value near or at zero. As a result, the strain affected to the patient's body by cross-currents is well controlled and much reduced if not eliminated.

It is to be noted that the amplitude of any cross-current that may remain can be influenced. To this end, a current monitor 67, in particular for monitoring cross-current, is provided which is connected to the sensor arrangements 38, 38' of either output socket 17, 17'. Using the measurement values, the current monitor configured to determine the cross-current flowing through the body of the patient. Said current monitor 67 co-operates with an automatic polarity switcher 69 of the common control unit 6. By switching polarity employing the polarity inverter 65 and comparing the cross-current flowing with or without the polarity inverter 65 engaged, it determined in which polarity stayed a lower cross-current results of. Accordingly, the automatic polarity switcher 69 sets the polarity inversion such that the lower cross-current results. As this can vary dynamically depending on the actual location at which the electrosurgical instruments 19 are used, the situation may change often and rapidly depending on variations of Rbody. The automatic polarity switcher 69 automatically keeps track of this and therefore provides a valuable boost of practicability of the dual activated electrosurgical instruments.

## Claims

1. Electrosurgical generator configured to output a high-frequency alternating voltage to an electrosurgical instrument (19), comprising an inverter unit (4) generating a high-frequency alternating voltage to be supplied to at least two output sockets (17, 17') for connection of electrosurgical instruments (19, 19'), wherein the inverter unit (4) is configured for simultaneous activation of the output sockets (17, 17') and the respective connected electrosurgical instruments,
**characterized in that**
the inverter unit (4) is a multilevel inverter and comprises a plurality of co-operating inverter cells (5-1, 5-2, 5-3, 5-4), said plurality of inverter cells being connected in a cascaded manner in a first state and being split into at least two groups (I, II) in a second state, each group being assigned one of the output sockets (17, 17') and feeding its generated high-frequency alternating output voltage via electrode lines to said assigned output socket,
wherein the inverter unit (4) is selectively switchable between first and second state upon direction and/or command.

2. Electrosurgical generator of claim 1, wherein the groups (I, II) with their respective inverter cells are being commonly controlled by synchronized reference signals (60, 60') being generated by a common control unit (6).

3. Electrosurgical generator of claim 1 or 2, wherein the reference signals (60, 60') are being synchronized such that the output voltages have the same frequency.

4. Electrosurgical generator of claim 3, wherein the output of one group (I) is synchronized in phase with a predefined phase shift to the output voltage of another group (II).

5. Electrosurgical generator of claim 4, wherein the predefined phase shift is zero such that the output voltages have the same phase angle.

6. Electrosurgical generator of claim 4, wherein one of the output voltages is inverted with respect to the output voltage of another group.

7. Electrosurgical generator according to any of the preceding claims, wherein at least two groups share one of the electrode lines, in particular they share a common neutral electrode (34), wherein preferably the output sockets (17, 17') are configured for unipolar electrosurgical instruments.

8. Electrosurgical generator according to any of the preceding claims 1 to 6, wherein each group has its own electrodes, in particular an active and neutral electrode (33, 34), neither of them being shared with another group, wherein preferably the output sockets (17, 17') are configured for bipolar electrosurgical instruments.

9. Electrosurgical generator according to the preceding claim, wherein a polarity of the output voltage of one of the groups (I, II) can be selectively changed, preferably by a polarity inverter (65).

10. Electrosurgical generator according to any of the preceding claims, wherein a current monitor (67) is provided which is configured to determine a cross-current between the output sockets (17, 17').

11. Electrosurgical generator according to the preceding claim, wherein an automatic polarity switcher (69) is provided for at least one of the groups, wherein the automatic polarity switcher (69) is configured to co-operate with the current monitor (67) for identifying a polarity having minimal cross-current.

12. Electrosurgical generator according to the preceding claim, wherein the automatic polarity switcher (69) is configured to co-operate with the current monitor (67) such as to (i) determine the cross-current in a first polarity state, (ii) then switch the polarity to a second polarity state and determine the cross-current in the second polarity state, (iii) comparing the cross-currents of the first and second polarity state, (iv) determine which polarity state has a lower cross-current, and (v) selecting the polarity having the lower cross-current.

13. Electrosurgical generator according to any of the preceding claims, wherein a beat frequency monitor (66) is provided that is configured to determine a beat frequency between outputted high-frequency voltages of the at least two output sockets (17, 17'), wherein preferably the beat frequency monitor (66) co-operates with a frequency following unit (62), preferably for ensuring frequency, and further preferably phase, synchronicity between the at least two groups (I, II).

14. Electrosurgical generator according to any of the preceding claims, wherein a decoupling control unit (68) is provided that is configured to automatically modify a power setting of one (II) of the group of the inverter cells upon a change of a power setting of the other group (I) of the inverter cells.

15. Electrosurgical generator according to any of the preceding claims, wherein a switching device (57) is provided which is configured to selectively alter an electrode, in particular a neutral electrode (34), between a shared and an unshared state.

## Patentansprüche

1. Elektrochirurgischer Generator, der dazu ausgelegt ist, eine hochfrequente Wechselspannung an ein elektrochirurgisches Instrument (19) auszugeben, umfassend eine Invertereinheit (4), die eine hochfrequente Wechselspannung erzeugt, welche an wenigstens zwei Ausgangsbuchsen (17, 17') zum Anschluss von elektrochirurgischen Instrumenten (19, 19') geliefert werden soll, wobei die Invertereinheit (4) für eine gleichzeitige Aktivierung der Ausgangsbuchsen (17, 17') und der jeweils angeschlossenen elektrochirurgischen Instrumente ausgelegt ist,
**dadurch gekennzeichnet, dass**
die Invertereinheit (4) ein Multilevel-Inverter ist und mehrere zusammenwirkende Inverterzellen (5-1, 5-2, 5-3, 5-4) umfasst, wobei die mehreren Inverterzellen in einem ersten Zustand kaskadiert verbunden sind und in einem zweiten Zustand in wenigstens zwei Gruppen (I, II) aufgeteilt sind, wobei jeder Gruppe eine der Ausgangsbuchsen zugewiesen (17, 17') ist und die jeweils erzeugte hochfrequente Wechselausgangsspannung über Elektrodenleitungen der zugewiesenen Ausgangsbuchse zugeführt wird,
wobei die Invertereinheit (4) auf Anweisung und/oder Befehl selektiv zwischen dem ersten und dem zweiten Zustand umschaltbar ist.

2. Elektrochirurgischer Generator nach Anspruch 1, wobei die Gruppen (I, II) mit den jeweils zugehörigen Inverterzellen gemeinsam durch synchronisierte Referenzsignale (60, 60') gesteuert werden, die durch eine gemeinsame Steuereinheit (6) generiert werden.

3. Elektrochirurgischer Generator nach Anspruch 1 oder 2, wobei die Referenzsignale (60, 60') dergestalt synchronisiert werden, dass die Ausgabespannungen dieselbe Frequenz aufweisen.

4. Elektrochirurgischer Generator nach Anspruch 3, wobei die Ausgabe einer Gruppe (I) mit einer vordefinierten Phasenverschiebung zu der Ausgabespannung einer anderen Gruppe (II) phasensynchronisiert ist.

5. Elektrochirurgischer Generator nach Anspruch 4, wobei die vordefinierte Phasenverschiebung gleich null ist, sodass die Ausgabespannungen denselben Phasenwinkel aufweisen.

6. Elektrochirurgischer Generator nach Anspruch 4, wobei eine der Ausgabespannungen im Hinblick auf die Ausgabespannung einer anderen Gruppe invertiert ist.

7. Elektrochirurgischer Generator gemäß einem der vorstehenden Ansprüche, wobei sich wenigstens zwei Gruppen eine der Elektrodenleitungen teilen, insbesondere teilen sie sich eine gemeinsame neutrale Elektrode (34), wobei die Ausgangsbuchsen (17, 17') vorzugsweise für unipolare elektrochirurgische Instrumente ausgelegt sind.

8. Elektrochirurgischer Generator gemäß einem der vorstehenden Ansprüche 1 bis 6, wobei jede Gruppe über ihre eigenen Elektroden verfügt, insbesondere eine aktive und eine neutrale Elektrode (33, 34), von denen keine mit einer anderen Gruppe geteilt wird, wobei die Ausgangsbuchsen (17, 17') vorzugsweise für bipolare elektrochirurgische Instrumente ausgelegt sind.

9. Elektrochirurgischer Generator gemäß dem vorstehenden Anspruch, wobei eine Polarität der Ausgangsspannung einer der Gruppen (I, II) selektiv geändert werden kann, vorzugsweise durch einen Polaritätsinverter (65).

10. Elektrochirurgischer Generator gemäß einem der vorstehenden Ansprüche, wobei eine Stromüberwachung (67) vorgesehen ist, die dazu ausgelegt ist, einen Querstrom zwischen den Ausgangsbuchsen (17, 17') zu bestimmen.

11. Elektrochirurgischer Generator gemäß dem vorstehenden Anspruch, wobei ein automatischer Polaritätsumschalter (69) für wenigstens eine der Gruppen vorgesehen ist, wobei der automatische Polaritätsumschalter (69) dazu ausgelegt ist, mit der Stromüberwachung (67) zusammenzuwirken, um eine Polarität zu identifizieren, die einen minimalen Querstrom aufweist.

12. Elektrochirurgischer Generator gemäß dem vorstehenden Anspruch, wobei der automatische Polaritätsumschalter (69) dazu ausgelegt ist, mit der Stromüberwachung (67) zusammenzuwirken, etwa um (i) den Querstrom in einem ersten Polaritätszustand zu bestimmen, (ii) die Polarität dann auf einen zweiten Polaritätszustand umzuschalten und den Querstrom in dem zweiten Polaritätszustand zu bestimmen, wobei (iii) die Querströme des ersten und des zweiten Polaritätszustands vergleichen werden, (iv) zu bestimmen, welcher Polaritätszustand einen geringeren Querstrom aufweist, und hierbei (v) die Polarität mit dem geringeren Querstrom auszuwählen.

13. Elektrochirurgischer Generator gemäß einem der vorstehenden Ansprüche, wobei eine Schwebungsfrequenzüberwachung (66) vorgesehen ist, die dazu ausgelegt ist, eine Schwebungsfrequenz zwischen ausgegebenen Hochfrequenzspannungen der wenigstens zwei Ausgangsbuchsen (17, 17') zu bestimmen, wobei die Schwebungsfrequenzüberwachung (66) vorzugsweise mit einer Frequenzfolgeeinheit (62) zusammenwirkt, vorzugsweise zum Sicherstellen einer Synchronizität der Frequenz, und ferner vorzugsweise der Phase, zwischen den wenigstens zwei Gruppen (I, II).

14. Elektrochirurgischer Generator gemäß einem der vorstehenden Ansprüche, wobei eine Entkopplungssteuereinheit (68) vorgesehen ist, die dazu ausgelegt ist, eine Leistungseinstellung einer Gruppe (II) der Inverterzellen bei einer Änderung einer Leistungseinstellung der anderen Gruppe (I) der Inverterzellen automatisch zu modifizieren.

15. Elektrochirurgischer Generator gemäß einem der vorstehenden Ansprüche, wobei eine Umschaltvorrichtung (57) vorgesehen ist, die dazu ausgelegt ist, eine Elektrode, insbesondere eine neutrale Elektrode (34), selektiv zwischen einem geteilten und einem ungeteilten Zustand zu wechseln.

## Revendications

1. Générateur électrochirurgical configuré pour sortir une tension alternative à haute fréquence vers un instrument électrochirurgical (19), comprenant une unité onduleur (4) générant une tension alternative à haute fréquence destinée à être fournie à au moins deux prises de sortie (17, 17') pour la connexion d'instruments électrochirurgicaux (19, 19'), dans lequel l'unité onduleur (4) est configurée pour l'activation simultanée des prises de sortie (17, 17') et des instruments électrochirurgicaux respectivement connectés,
**caractérisé en ce que**
l'unité onduleur (4) est un onduleur à plusieurs niveaux et comprend une pluralité de cellules d'onduleur coopérantes (5-1, 5-2, 5-3, 5-4), ladite pluralité de cellules d'onduleur étant connectée en cascade dans un premier état et étant divisée en au moins deux groupes (I, II) dans un second état, chaque groupe étant affecté à l'une des prises de sortie (17, 17') et introduisant sa tension de sortie alternative à haute fréquence générée par le biais de lignes d'électrodes dans ladite prise de sortie affectée,
dans lequel l'unité onduleur (4) peut être commutée de manière sélective entre un premier et un second état sur instruction et/ou commande.

2. Générateur électrochirurgical selon la revendication 1, dans lequel les groupes (I, II) avec leurs cellules d'onduleur respectives sont commandés en commun par des signaux de référence synchronisés (60, 60') étant générés par une unité de commande commune (6).

3. Générateur électrochirurgical selon la revendication 1 ou 2, dans lequel les signaux de référence (60, 60') sont synchronisés de telle sorte que les tensions de sortie aient la même fréquence.

4. Générateur électrochirurgical selon la revendication 3, dans lequel la sortie d'un groupe (I) est synchronisée en phase avec un déphasage prédéfini par rapport à la tension de sortie d'un autre groupe (II).

5. Générateur électrochirurgical selon la revendication 4, dans lequel le déphasage prédéfini est nul de telle sorte que les tensions de sortie aient le même angle de phase.

6. Générateur électrochirurgical selon la revendication 4, dans lequel l'une des tensions de sortie est inversée par rapport à la tension de sortie d'un autre groupe.

7. Générateur électrochirurgical selon l'une quelconque des revendications précédentes, dans lequel au moins deux groupes se partagent l'une des lignes d'électrodes, en particulier ils se partagent une électrode neutre commune (34), dans lequel de préférence les prises de sortie (17, 17') sont configurées pour des instruments électrochirurgicaux unipolaires.

8. Générateur électrochirurgical selon l'une quelconque des revendications 1 à 6 précédentes, dans lequel chaque groupe possède ses propres électrodes, en particulier une électrode active et une électrode neutre (33, 34), aucune d'entre elles n'étant partagée avec un autre groupe, dans lequel de préférence les prises de sortie (17, 17') sont configurées pour des instruments électrochirurgicaux bipolaires.

9. Générateur électrochirurgical selon la revendication précédente, dans lequel une polarité de la tension de sortie de l'un des groupes (I, II) peut être modifiée de manière sélective, de préférence par un inverseur de polarité (65).

10. Générateur électrochirurgical selon l'une quelconque des revendications précédentes, dans lequel un dispositif de surveillance de courant (67) qui est fourni est configuré pour déterminer un courant transversal entre les prises de sortie (17, 17').

11. Générateur électrochirurgical selon la revendication précédente, dans lequel un commutateur de polarité automatique (69) est fourni pour au moins un des groupes, dans lequel le commutateur de polarité automatique (69) est configuré pour coopérer avec le dispositif de surveillance de courant (67) pour identifier une polarité ayant un courant transversal minimal.

12. Générateur électrochirurgical selon la revendication précédente, dans lequel le commutateur automatique de polarité (69) est configuré pour coopérer avec le dispositif de surveillance de courant (67) de manière à (i) déterminer le courant transversal dans un premier état de polarité, (ii) commuter ensuite la polarité vers un second état de polarité et déterminer le courant transversal dans le second état de polarité, (iii) comparer les courants transversaux des premier et second états de polarité, (iv) déterminer quel état de polarité présente un courant transversal plus faible, et (v) sélectionner la polarité présentant le courant transversal le plus faible.

13. Générateur électrochirurgical selon l'une quelconque des revendications précédentes, dans lequel un dispositif de surveillance de fréquence de battement (66) qui est fourni est configuré pour déterminer une fréquence de battement entre des tensions à haute fréquence délivrées des au moins deux prises de sortie (17, 17'), dans lequel le dispositif de surveillance de fréquence de battement (66) coopère de préférence avec une unité de suivi de fréquence (62), de préférence pour assurer la synchronisation en fréquence, et de préférence en outre en phase, entre les au moins deux groupes (I, II).

14. Générateur électrochirurgical selon l'une quelconque des revendications précédentes, dans lequel une unité de commande de découplage (68) qui est fournie est configurée pour modifier automatiquement un réglage de puissance d'un (II) du groupe de cellules d'onduleur lors d'une modification d'un réglage de puissance de l'autre groupe (I) des cellules d'onduleur.

15. Générateur électrochirurgical selon l'une quelconque des revendications précédentes, dans lequel un dispositif de commutation (57) qui est fourni est configuré pour modifier sélectivement une électrode, en particulier une électrode neutre (34), entre un état partagé et un état non partagé.
